# EUROPEAN PATENT APPLICATION

(11) **EP 4 218 577 A1**
(43) Date of publication of application: **02.08.2023**
(21) Application number: 22153579.2
(22) Date of filing: 27.01.2022
(51) Int. Cl.: A61B 5/16, A61B 5/316, G16H 50/20, A61B 5/055, A61B 5/00, G01R 33/48

(54) **A METHOD AND SYSTEM FOR ASSESSING A SUBJECT**

(71) Applicant: Koninklijke Philips N.V., 5656 AG Eindhoven (NL)
(72) Inventor: LEUFKENS, Timmy Robertus, Eindhoven (NL); HART DE RUIJTER-BEKKER, Evelijne Machteld, Eindhoven (NL); KLAMING, Laura, Eindhoven (NL); VAN EE, Raymond, Eindhoven (NL); MENA BENITO, Maria Estrella, Eindhoven (NL)
(74) Representative: Philips Intellectual Property & Standards

(57) **Abstract**

A method and system for assessing a subject using fMRI analysis of the brain activity of a subject while they perform a set of cognitive tasks. For each task, a first level of performance in completing the task is derived. For at least some of the tasks which have been completed to a satisfactory level of performance, it is assessed if any of those tasks showed increased activity, above a threshold, of a region of the brain different to a region of the brain associated with the task. For those tasks, a further fMRI analysis is carried out while they perform a further cognitive task but with additional cognitive loading. The fMRI analysis and the further fMRI analysis are compared. This for example enables determination that a patient is using compensation mechanisms to complete the cognitive tasks.

## Description

### FIELD OF THE INVENTION

The invention relates to a method and system for assessing a subject, and in particular to assess mild traumatic brain injury.

### BACKGROUND OF THE INVENTION

Mild traumatic brain injury (mTBI) can be described as a traumatically induced physiological disruption of brain function manifested by at least one of the following:
any period of loss of consciousness;
any loss of memory for events immediately before or after the accident;
any alteration in mental state at the time of the accident; and
focal neurological deficit(s) that may or may not be transient.

Traumatic brain injury (TBI) and in particular mTBI constitutes a large and rapidly growing public health concern. Each year, around 69 million people worldwide sustain a TBI. About 85% of these TBIs are diagnosed as mTBI.

Most mTBI patients develop a set of symptoms that include headache, dizziness, fatigue, vertigo, neuropsychiatric symptoms (including behavioral and mood changes, confusion), difficulty in balancing, changes in sleep patterns, and cognitive impairments (including memory, attention, concentration, and executive function disorders).

In the acute phase, current diagnosis of mTBI is based on a clinical assessment of mental state changes (for example using the Glasgow Coma Scale). A cognitive assessment may be conducted at a later stage, in patients with chronic cognitive symptoms.

The main cognitive functions that are impaired in mTBI are attention and executive functioning (e.g. working memory, processing speed). In addition, mTBI patients often have visual and balance problems that may worsen their cognitive deficits. For an evaluation of a patient with mTBI, thorough assessment of attention, processing speed, executive functions, and memory is necessary to capture any current cognitive deficits. More importantly, since symptoms are often subtle, these tests need to have high sensitivity.

Standard neuropsychological and cognitive tests that are used in clinical practice for this purpose are not sensitive enough to detect these subtle cognitive deficits. These standard tests are often characterized by: absence of multitasking; no distraction in the lab; tasks that are too easy; a duration of tasks that is too short; an inadequate choice of tasks (e.g., presenting a memory task while the patient's memory problems are more likely due to attentional deficits); patients putting more effort in the performance of the lab task as compared to tasks in daily life; and compensatory mechanisms being used during the lab task.

As a result, despite the fact that there has been an increasing recognition of the incidence of mTBI, the current assessment criteria for mTBI remain suboptimal and hence do not have the required sensitivity. This is mainly because the current neuropsychological and cognitive assessment use a standard methodology for all patients. As a result, the subtle cognitive impairment that mTBI patients encounter in their daily life may not be revealed during the clinical assessment. Cognitive impairment in mTBI is characterized by subtle cognitive deficits that impair normal everyday functioning and as a consequence limit mTBI patients in their ability to have a normal social and work life.

The insufficient sensitivity of the current tests means that when cognitive deficiencies in mTBI patients are assessed, the cognitive test scores are often within the norm, i.e. indistinguishable from those of healthy individuals. As a result, a high percentage of mTBI patients may not receive an accurate diagnosis and as a consequence may not receive any treatment.

In other words, despite mTBI patients complaining of cognitive difficulties in their daily lives, their problems are not detected by the current ways of assessing cognitive deficits in mTBI.

### SUMMARY OF THE INVENTION

The invention is defined by the claims.

According to examples in accordance with an aspect of the invention, there is provided a method of assessing a subject, comprising:
performing a functional MRI, fMRI, analysis of the brain activity of a subject while they perform a set of cognitive tasks, wherein each cognitive task is associated with activity of a particular region or network of regions of the brain;
for each task, receiving an indication of, or identifying, a first level of performance in completing the task;
for at least some of the tasks which have been completed to a threshold level of performance, identifying any tasks for which there is increased activity, above a threshold, of a different region or network of regions of the brain to the region or network of regions of the brain associated with the task;
for the identified tasks, performing a further fMRI, analysis of the brain activity of the subject while they perform a further cognitive task associated with activity of the same region or network or regions of the brain as the identified task, but with additional cognitive loading provided to the subject; and
for each further task, receiving an indication of, or identifying, a second level of performance in completing the task for comparison with the first level of performance.

This method addresses the problem that subjects (i.e. patients) are usually examined using cognitive tests and are often underdiagnosed because the cognitive tests are not able to detect the subtle impairments in cognitive functioning.

The method provides a two stage approach enabling subtle impairments to be detected, by first letting a patient perform a number of cognitive tasks covering most cognitive domains in an MR scanner. The analysis of the MR scan data looks at the expected brain areas and networks associated with each task, that normally should become activated while performing the particular certain task (i.e. there is a task A for testing for cognitive domain A which task is designed to activate brain areas related to domain A).

If the brain area and network related to cognitive domain A is activated (as detected by fMRI) during performance of the cognitive task for cognitive domain A, and the task is performed to a satisfactory level then there is no issue in that cognitive domain and its related functional areas.

If conversely the related brain areas and networks are not activated as expected, but the patient still performs to a satisfactory level, i.e. without serious performance decline, it is likely that the patient has affected brain areas, but the brain is using other areas and networks in order to still be able to perform the task. Thus, the brain is making use of reserve capacity. This type of response is typically missed, whereas the method of the invention still enables the brain impairment to be detected.

In particular, if the expected brain areas related to a cognitive domain, e.g. cognitive domain A, are not activated while performing cognitive task related to cognitive domain A, but the behavioral measures are still within expected values, the subject is then provided with added workload. This may exhaust the (other) cognitive resources and this provides an indication of subtle cognitive impairments in the particular cognitive domain.

If there is still no significant decrease in performance, it can be concluded that the patient is able to perform well, but uses cognitive reserves. This may lead to problems in the long run. If there is a significant decrease in performance (compared to the test of the same cognitive domain in the lower workload test), it can be concluded that the subject has a cognitive problem in the specified cognitive domain and a treatment and care path can be tailored accordingly.

The method may comprise, for each task which has been completed to a threshold level of performance, determining that activity of the particular region or network of regions associated with the task is below an expected level, and only then identifying said any tasks for which there is increased activity.

Decreased activity or even absence of activity at an expected region or network is an indication of brain trauma as explained above. It indicates suboptimal or non-functional regions or networks and already provide insights that the cognitive domain related to the task is affected at least anatomically. The increase of activity at a different region or network is then a further second indication of affected cognitive domain.

The cognitive tasks for example include one or more of executive function tasks, attention tasks, planning tasks, and memory tasks.

The cognitive tasks for example include one or more of vigilance tasks, the Flanker task and the Posner cueing task.

Various possible additional cognitive loading methods may be used. For example, the additional cognitive loading may comprise one or both of oculomotor and pupillometric loading.

In another example, the additional cognitive loading comprises Autonomic Nervous System (ANS) regulatory loading.

Multiple ANS functions also are known to be susceptible to mTBI-related dysregulation. For instance, it has been shown that mTBI reduces cerebrovascular reactivity and mTBI individuals have shown higher heart rate, decreased heart rate variability and reduced skin conductance in comparison to healthy individuals.

A method to provoke ANS functions is to induce hypoxia (a reversible, temporary decrease of oxygen levels). Measures of ANS function are sensitive to hypoxia, as the literature indicates the existence of differences in pulse oximetry, aspects of cerebrovascular regulatory responses, heart rate, electrodermal activity and the cardiovascular response.

The affected ANS functions in turn are known to have a negative effect on cognitive functions and disproportionally more in mTBI patients.

In another example, the additional cognitive loading comprises additional tests that engage working memory or attention.

In another example, the additional cognitive loading comprises additional time constraints on tasks, degraded stimuli quality, or increased frequency of stimuli.

In all cases, the level of the additional cognitive load may be variable based on a level of brain activity in real time obtained by fMRI analysis. Thus, the additional load may be adapted using feedback from the brain activity analysis.

The method for example further comprising monitoring vital signs of the subject. Some vital signs may be used as a measure of cognitive effort and indicative of patients with mTBI.

The method may comprise using eye tracking to assess cognitive load.

The invention also provides a computer program comprising computer program code which is adapted, when said program is run on a computer, to implement the method defined above.

The invention also provides a processor which is programed with the computer program. The invention also provides a system for assessing cognitive performance, comprising:
an input for receiving fMRI images of the brain activity of a subject while they perform a set of cognitive tasks;
the processor as defined above for controlling an output device for delivering tasks to the subject and for processing the fMRI images.

The invention also provides an MRI system comprising:
a fMRI scanner;
an output device for delivering tasks to a subject; and
the system for assessing cognitive performance as defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an MRI system; and
Fig. 2 shows a method of assessing a subject.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method and system for assessing a subject which uses fMRI analysis of the brain activity of a subject while they perform a set of cognitive tasks. For each task, a first level of performance in completing the task is derived. For at least some of the tasks which have been completed to a satisfactory level of performance, it is assessed if any of those tasks showed increased activity, above a threshold, of region of the brain different to a region of the brain associated with the task. For those tasks, a further fMRI analysis is carried out while they perform a further cognitive task but with additional cognitive loading. The fMRI analysis and the further fMRI analysis are compared. This for example enables determination that a patient is using compensation mechanisms to complete the cognitive tasks.

The approach of the invention may be considered to be analogous to a cardiac Vmax test (e.g. a bicycle test for testing maximum physical capabilities). The additional cognitive loading functions as an additional cognitive stress that challenges the patient in a way that resembles stress during daily routines by requiring extra effort in the form of extra cognitive load, or resource depletion. The invention may in this way provide an effective method for uncovering latent or hidden brain damage and associated cognitive impairment in mTBI.

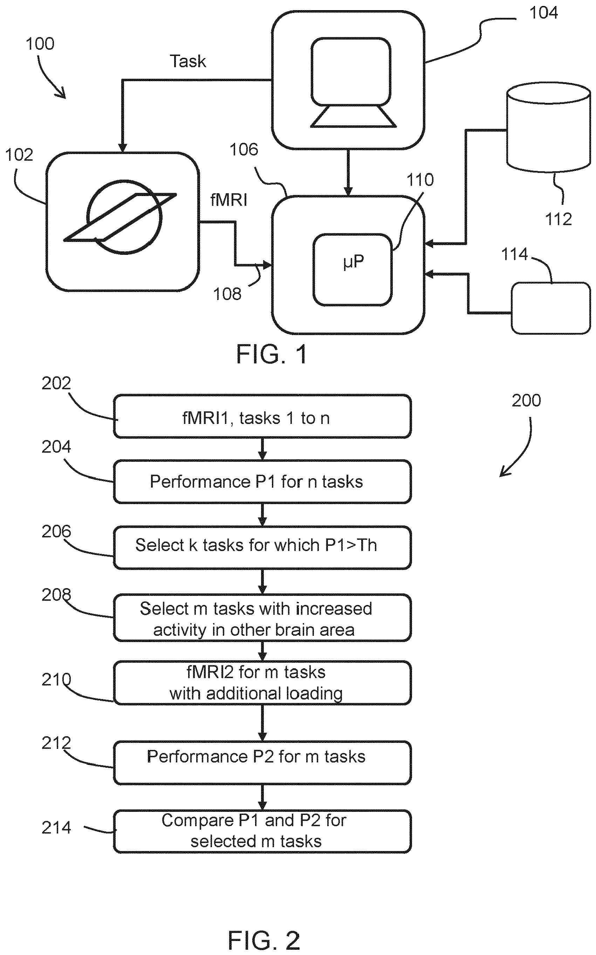

Fig. 1 shows an MRI system 100 comprising a fMRI scanner 102, and an output device 104 delivering tasks to a subject. The fMRI scanner comprises an advanced functional MR Imaging unit and a fMRI scan unit.

The output device 104 is for example a display screen or virtual reality system to provide visual stimuli, integrated in the MR bore.

A system 106 is provided for assessing cognitive performance, comprising an input 108 for receiving fMRI images of the brain activity of a subject while they perform the tasks delivered to the subject and a processor 110. The processor 110 performs analysis of the fMRI images and also determines (or is advised of) the level of performance of the subject in conducting the tasks. This performance information may be determined by the system 106. For example, the subject may provide input to the system as part of the cognitive task (e.g. via speech, using a keyboard, via a touch sensitive input, or any other user interface device) or the system may determine the performance based on information gathered using sensors.

Alternatively, a clinician may input performance results into the system 106 manually.

The processor for example determines the behavioral response to the cognitive test stimuli under variable cognitive load levels and then assesses the cognitive performance data according to predetermined quality rules.

The processor 110 also controls the output device 104 for delivering tasks to the subject.

A database 112 is provided of cognitive tests to be applied to the subject. A sensor arrangement 114 is also shown, for example comprising a system for making oculomotor measurements, and/or a system for making pupillometry measurements and/or vital signs sensors e.g. for blood pressure monitoring;

In use of the system, a clinician chooses tests to be applied. The system for example provides guidance for this purpose.

The cognitive effort required by the subject, i.e. the additional effort compared to their previous rest state, is assessed from the analysis of the fMRI images while the subject performs each cognitive task. In this way, brain areas (i.e. a particular region or network of regions of the brain) are identified that are stimulated by the particular task. The term "brain area" will be used to denote a single region or network of regions of the brain, and optionally also the switching mechanism between neuronal networks. Indeed, fMRI studies suggest a superordinate cognitive control network, comprising the dorsal regions of the lateral prefrontal cortex (DLPFC), anterior cingulate cortex (dACC) and parietal cortex (DPC).

Different brain areas are associated with different types of cognitive task. Thus, each task has an expected brain area that will be stimulated during performance of the task.

A discussion of different cognitive domains, related cognitive tasks, related functional neurological structures, and additional cognitive loading can be found in MJ Cook et. al., Task-related functional magnetic resonance imaging activation in patients with acute and subacute mild traumatic brain injury: A coordinate-based meta-analysis, Neuroimage Clin. 2020;25;102129.

If a subject is not able to complete a task to an expected level of performance, this is an indication of possible mTBI, as detected by conventional fMRI based cognitive testing. However, if the task is completed to an expected level of performance, the invention provides a further analysis to determine if there may still be a cognitive impairment, even for the brain area associated with the task which has been successfully completed. In particular, the invention enables brain areas to be identified that are used for compensation, in order to execute the cognitive task.

Fig. 2 shows the method used.

The method 200 comprises in step 202 receiving first fMRI images, and performing fMRI analysis of the brain activity of a subject while they perform a set (1 to n) of cognitive tasks. Each cognitive task is associated with activity of a particular area of the brain.

In step 204, for each task 1 to n, a first level of performance P1 is obtained in completing the task. This performance may be sensed (e.g. by user movement, or gaze, or voice/sound recognition) or it may be input by a clinician, or it may derived from user input (e.g. by touching a screen to follow commands of the cognitive task).

Some tasks, e.g. a number k of those n tasks (k<=n) , will be performed to a satisfactory level. Those tasks are identified in step 206. For some of those k tasks which have been completed to a threshold level of performance, there will be a sub-set of tasks e.g. a number m of those tasks (m<=k) for which the fMRI analysis shows an increased activity, above a threshold, of a different area of the brain to the area the brain associated with the task. These m tasks are identified in step 208.

For the identified tasks, a further (second) fMRI analysis of the brain activity of the subject is carried out in step 210 while they perform a further cognitive task associated with activity of the same brain area as the identified task, but with additional cognitive loading provided. This additional cognitive loading may be of the same type as the original task (but requiring additional brain resource) so that an increased amount of compensation is needed to perform the task as a result of the additional cognitive loading. However, the cognitive loading may be implemented as a different type of task which is for example aimed at the different brain area which has been identified as being used for compensation. In that case, the additional cognitive loading has an impact on the ability of the subject to compensate and this will again result in differences in performance which can be analyzed.

For each further task, a second level of performance P2 in completing the task is obtained. There is a comparison in step 214 between the first and second levels of performance of pairs of tasks (i.e. tasks targeting the same brain area, with and without additional cognitive loading).

When the k tasks are identified in step 206, which were completed to the threshold level of performance, a further selection may be made before the selection in step 208, to determine that the activity of the particular brain area is below an expected level. Thus, only if the associated brain area is less stimulated than expected, will tasks then be selected to which additional cognitive loading is to be applied.

The method thus provides a safe, protocolized, in bore, real-time fMRI, multimodal 'stress' module. The additional cognitive loading means the cognitive, oculomotor and pupillometric, and ANS regulatory capacities may be fully exhausted. This may then provoke cognitive deficits which are normally hidden. The ANS regulatory capacities for example include the heart rate and electrodermal activity.

The additional cognitive loading gives rise to lowered performance on cognitive tests as compared to both non-stress situations and healthy individuals. It is of particular interest when the fMRI activity indicates a lack of normal increases in the brain area expected to be involved in cognitive control, as explained above. Cognitive control is the process of employing executive functions, such as attention, planning or working memory, to guide appropriate behaviors in order to achieve a specific goal.

The tests to assess cognitive performance can include any standard neuropsychological test, such as tests of executive function and attention. Ideally, cognitive tests from experimental neuropsychology may be used which are better suited to measure subtle cognitive impairment, e.g. tests like vigilance tasks, the Flanker task or the Posner cueing task.

The additional cognitive loading may be provided by adding features or tests that engage working memory or attention. This can be accomplished by increasing the time pressure of completing the task, by degrading the stimuli, by decreasing the inter-stimulus-interval, by adding positive or negative reinforcement, by adding multiple tasks, by increasing the number of distracting stimuli, or by increasing the duration of the task.

The responses to the cognitive tests may be collected under a variable additional cognitive load level. Cognitive load levels can in this way be adapted based on real-time performance (e.g. using a working memory task).

One example will now be presented in more detail.

This example is based on testing the working memory cognitive domain. Working memory is the ability to transiently store and manipulate information to be used for cognitive or behavioral guidance. Previous functional MR imaging studies in healthy subjects have shown that increased working memory load is associated with increased activation of the bilateral frontal and parietal regions. These regions are known to be vulnerable to damage in TBI. Malfunctioning of working memory has been frequently reported in TBI.

As an example of a suitable cognitive task, the n-back task is a continuous performance task that is commonly used to measure working memory. In an n-back task, the subject is presented with a sequence of stimuli, and the task consists of indicating when the current stimulus matches the one from n steps earlier in the sequence. The load factor n can be adjusted to make the task more or less difficult. This can be regarded as control of an additional degree of cognitive loading.

For example, an auditory three-back test could consist of the system delivering, or an experimenter reading, the following list of letters to the test subject:
TLHCHOCQLCKLHCQTRRKCHR

The subject is supposed to respond with a confirmation when the letters marked in bold and underlined are read, because those correspond to the letters that were read three steps earlier.

The n-back task captures the active part of working memory. When n equals 2 or more, it is not enough to simply keep a representation of recently presented items in mind; the working memory buffer also needs to be updated continuously to keep track of what the current stimulus must be compared to. To accomplish this task, the subject needs to both maintain and manipulate information in working memory.

In healthy subjects, increased activation is found in the bilateral frontal and parietal regions consistent with activation of working memory circuitry. After increased working memory load (i.e. increasing the value of n), an increase in brain activation in response to each increase in working memory load will be detected.

In mTBI patients there may be hardly any difference detected in neurological activation in the specific brain regions between three-back versus two-back. This for example provides an indication of the situation that the maximum (or a plateau) has been reached in these patients and that they cannot use those brain regions anymore and need to revert to other regions (i.e. compensate).

An example of additional cognitive could be to present a dual-task n-back task: in the dual-task paradigm, two independent sequences are presented simultaneously, typically using different modalities of stimuli, such as one auditory and one visual. Thus, one task may use the same brain area as the original task, but the added task uses other brain resource.

Adding even more additional workload could be to distort the quality of the visual stimuli increasing the effort to read the letters.

More generally, additional cognitive loading can be achieved by transitioning from any single-mode stimuli to any multimodal stimuli (e.g. visual and auditory stimuli) presented simultaneously to the patient. Visual and auditory stimuli may however be presented either congruently or non-congruently. Non-congruent stimulation asks for increased attentional resources.

As explained above, the aim is to detect whether a cognitive load activates other brain areas than those that are usually expected to be activated while performing a cognitive task. For the example of the n-back task, the 'normal' test level may not show a difference in performance and neuronal activity between healthy subjects and patients with mTBI.

However, when adding cognitive load, in healthy subjects it may be expected that similar regions will be activated, but if the regions are (subtly) damaged, but not enough to show cognitive decline with the 'normal' level task, it may be expected that when cognitive load is increased other regions than the ones that may be expected will be activated. This indicates that the patient had to revert to cognitive reserve regions.

An example of a region that could be activated as compensation is the anterior cingulate, i.e. more attention-related regions.

The cognitive load may be measured using eye-tracking (e.g. saccadic response times) which is known to be sensitive to cognitive load.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A method of assessing a subject, comprising:
(202) performing a functional MRI, fMRI, analysis of the brain activity of a subject while they perform a set of cognitive tasks, wherein each cognitive task is associated with activity of a particular region or network of regions of the brain;
(204) for each task, receiving an indication of, or identifying, a first level of performance in completing the task;
(208) for at least some of the tasks which have been completed to a threshold level of performance, identifying any tasks for which there is increased activity, above a threshold, of a different region or network of regions of the brain to the region or network of regions of the brain associated with the task;
(210) for the identified tasks, performing a further fMRI, analysis of the brain activity of the subject while they perform a further cognitive task associated with activity of the same region or network or regions of the brain as the identified task, but with additional cognitive loading provided to the subject; and
(212) for each further cognitive task, receiving an indication of, or identifying, a second level of performance in completing the task for comparison with the first level of performance.

2. The method of claim 1, comprising, for each task which has been completed to a threshold level of performance, determining that activity of the particular region or network of regions associated with the task is below an expected level, and only then identifying said any tasks for which there is increased activity.

3. The method of claim 1 or 2, wherein the cognitive tasks include one or more of executive function tasks, attention tasks, planning tasks, and memory tasks.

4. The method of claim 3, wherein the cognitive tasks include one or more of vigilance tasks, the Flanker task and the Posner cueing task.

5. The method of any one of claims 1 to 4, wherein the additional cognitive loading comprises one or both of oculomotor and pupillometric loading.

6. The method of any one of claims 1 to 5, wherein the additional cognitive loading comprises Autonomic Nervous System (ANS) regulatory loading.

7. The method of any one of claims 1 to 6, wherein the additional cognitive loading comprises additional tests that engage working memory or attention.

8. The method of any one of claims 1 to 7, wherein the additional cognitive loading comprises additional time constraints on tasks, degraded stimuli quality, or increased frequency of stimuli.

9. The method of any one of claims 1 to 8, wherein the additional cognitive load is variable based on a level of brain activity in real time obtained by fMRI analysis.

10. The method of any one of claims 1 to 9, further comprising monitoring vital signs of the subject.

11. The method of any one of claims 1 to 10, further comprising using eye tracking to assess cognitive load.

12. A computer program comprising computer program code which is adapted, when said program is run on a computer, to implement the method of any one of claims 1 to 11.

13. A processor (110) which is programed with the computer program of claim 12.

14. A system (106) for assessing cognitive performance, comprising:
an input (108) for receiving fMRI images of the brain activity of a subject while they perform a set of cognitive tasks;
the processor (110) of claim 13 for controlling an output device for delivering tasks to the subject and for processing the fMRI images.

15. An MRI system comprising:
a fMRI scanner (102);
an output device (104) for delivering tasks to a subject; and
the system (106) for assessing cognitive performance of claim 14.
